# EUROPEAN PATENT APPLICATION

(11) **EP 1 604 704 A1**
(43) Date of publication of application: **14.12.2005**
(21) Application number: 05253500.2
(22) Date of filing: 07.06.2005
(51) Int. Cl.: A61M 29/02, A61M 25/10

(54) **Drug delivery device using microprojections**

(30) Priority: 08.06.2004 US 863044
(71) Applicant: Cordis Corporation, Miami Lakes, Florida 33014 (US)
(72) Inventor: Krulevitch, Peter, Pleasanton CA 94566 (US); Maghribi, Mariam, Livermore CA 94550 (US)
(74) Representative: Mercer, Christopher Paul

(57) **Abstract**

A device for delivering a therapeutic agent into tissue includes: an elongated body; and a first balloon in the body expandable from a collapsed position to an expanded position; and a second balloon in the body expandable from a collapsed position to an expanded position. The second balloon has a plurality of apertures therein. A therapeutic agent is located in the second balloon. A plurality of microprojections are located on a surface of the second balloon for penetrating tissue. Upon expansion of the first balloon from the collapsed position to the expanded position, the second balloon is expanded from the collapsed position to the expanded position for deploying the plurality of microprojections into tissue and for dispensing the therapeutic agent from the second balloon into the tissue through the plurality of apertures.

## Description

### FIELD AND BACKGROUND OF THE INVENTION

The present invention relates, in general, to drug delivery devices and methods for delivering drugs, and more particularly, to new and useful devices and methods for delivering drugs to tissue, for instance, for delivering drugs to a desired layer within tissue such as the adventitial layer of a vessel.

Obstructive atherosclerotic disease is a serious health problem facing our society today. This disease is the result of the deposit of fatty substances and cells and connective tissue on the interior of the walls of the arteries. The build-up or accumulation of such deposits results in a narrowing of the inside diameter of the artery which in turn restricts the blood flow through the artery. This disease, wherein the opening or lumen of the artery is narrowed, is known as atherosclerosis and the accumulation is known as a lesion.

One commonly used procedure for treating an obstruction caused by atherosclerosis is a procedure known as coronary artery bypass graft surgery ("bypass surgery"). Although bypass surgery has been used with moderate success in the treatment of atherosclerosis, it is invasive and traumatic to the patient.

One less invasive and traumatic procedure developed more recently is coronary angioplasty. Coronary angioplasty, and angioplasty in general, is a procedure in which a balloon is positioned in the inside of the artery at the site of the accumulation or lesion and inflated in order to dilate the atherosclerotic lesion and thus open the restricted area of the artery. In order to advance the balloon to the lesion, the balloon is attached to the distal end of a small diameter catheter, which includes means for inflating the balloon from the other end of the catheter. The catheter is maneuvered or "steered" through the patient's vessels to the site of the lesion with the balloon in an un-inflated form. When the un-inflated balloon is properly positioned at the lesion, the balloon is then inflated to dilate the restricted area.

While angioplasty has been relatively successful in treating coronary artery disease, restenosis of the treated site often occurs approximately 3 to 6 months following the procedure. It is believed that the primary factor in developing restenosis is the healing that takes place after the injury caused by the intervention of balloon dilation procedure. The restenosis has close analogy to scar formation following vascular surgery in that the histologic result has a similar morphology. The histologic response is called myointimal hyperplasia. The process of myointimal hyperplasia consists of the migration of smooth muscle cells through the internal elastic lamina into the vessel lumen where they then proliferate. The net result is a thickening of the vessel wall. Over time, this thickening re-occludes or re-stenosis the vessel to a point where it is clinically significant. That is, the blood flow through the vessel is diminished to a rate similar to the rate before the angioplasty procedure. The occurrence of this seems to happen approximately 30-35% of the time following an angioplasty to that specific site in coronary arteries.

Several alternative procedures have been attempted to try to affect the occurrence or rate of the restenosis following intervention to the lesion site in the coronary artery. These procedures have included the use of lasers, mechanical atherectomy devices, heated balloons, and metal implantable stents. While each of these procedures has shown some success in dealing with the initial lesion, all have the similar problem of restenosis at a similar or even greater occurrence. Current estimates of restenosis of the lesion site using these alternative procedures ranges between 40-50%. The time frame of restenosis of all of these is generally from 3-6 months after the procedure.

Therefore, it appears that this re-stenotic healing lesion area is independent of the type of interventional procedure used. Rather, it is a physiologic response to any type of injury brought to that lesion site. Because of this intervention independent physiologic response, it is felt by many physicians that potentially the best way to deal with restenosis would be by a pharmacologic means, such as a drug agent, targeted at the biochemical events that take place after injury.

To date, most pharmacologic trials involve either an oral or intravenously injected drug that is delivered throughout the whole body in hopes of trying to affect this small site in the arteries. This type of pharmacologic treatment is known as a "systemic treatment." Some agents that have been tried in human clinicals include: heparin, calcium channel blockers, angiotensin converting enzyme inhibitors, Omega-3 fatty acids, and growth peptides. Other agents that may not have been tried in clinicals but are of interest include thromboxane synthetase inhibitor, serotonin, growth factor inhibitors, growth factor analogs such as angiopeptin, antagonists, HMGCoA reductase inhibitors, platelet derived growth factor, inflammatory cell factors, platelet aggregation inhibitors, and thrombin inhibitors such as hirudin or its analogs.

The indication for use of most of these has been either in vitro-cell culture studies or animal studies. These studies have shown some effect on the smooth muscle cell proliferation and migration which are major components of the myointimal hyperplasia that takes place in the restenotic lesion. However, none of the systemic drug delivery human trials to date has shown a major effect on the occurrence of restenosis.

Even though none of these agents have been completely successful in the in-vivo human clinical trials, it is still generally felt that one of these agents or some other new agent, if delivered locally and site specifically to the lesion, would still be able to reduce the proliferative response. One of the problems with systemic techniques is the inability to deliver a high enough concentration of the agent locally at the lesion in order to affect the physiologic response. In the in-vitro and in-vivo animal studies which have shown some success, a high concentration of the agent was used. Thus, it is believed that if the agent was delivered specifically to the site as opposed to systemically, the agent may be delivered at a high enough concentration to truly affect the physiologic response.

The reason many of these agents have not been used in a higher concentration in-vivo in humans is that many of the agents may exhibit undesirable side effects. Thus, if a high concentration of the agents is given systemically, they may have unwanted physiologic effects. Therefore, if the drug can be given with high concentrations locally to the vessel wall while minimizing the systemic amount of drug, the desired result of modulating the restenotic growth while preventing any unwanted systemic effects may be achieved.

There are other ways known to date in trying to create a site specific local delivery of drug to a site. One approach presently contemplated is the use of a perforated or sweating balloon. For example, a drug delivery device is disclosed by Wolinsky, H., et al. in the article entitled, Use of a Perforated Balloon Catheter to Deliver Concentrated Heparin Into the Wall of a Normal Canine Artery, 15 JACC 475 (Feb. 1990). This device is a percutaneous transluminal coronary angioplasty (PTCA) balloon with several microholes in the balloon for delivery of an agent during balloon dilatation. The drug is incorporated into the same fluid which is used to inflate the balloon.

A disadvantage of available devices, such as the one disclosed by Wolinsky et al., is that these devices cause a substantial blockage of blood flow in the subject vessel during the procedure. Thus, such devices may only be used for the fairly short time frame (typically, from one to two minutes), similar to the time frame of the actual angioplasty dilatation.

Other available drug delivery devices are disclosed, for example, in U.S. Pat. No. 4,824,436 (Wolinsky) and U.S. Pat. No. 4,636,195 (Wolinsky). These devices are directed to a dual occlusion catheter in which a balloon is inflated proximally and distally of the accumulation or lesion creating a space for infusion of a drug. This dual balloon catheter creates a space for infusion of drug separate from the blood flow. This device, however, also can only be used for a short period of time because it occludes blood flow.

In these types of devices where a balloon is inflated inside the vessel, some means for providing perfusion through the catheter itself becomes important. It is necessary in such devices that the device provide a large latitude in time over which the agent could be delivered. Devices which occlude blood flow may not provide the necessary latitude. Because the basic research into the biochemistry and physiologic events indicate that the initial events begin immediately after injury and continue intensely for several hours, it is desirable for the drug delivery system to allow drug delivery for several hours to a day or two beginning immediately after intervention. This research also points out that the initial events subsequently create a cascade of events that ultimately lead to intimal thickening. While these accumulations or lesions do not become apparent for several months, it is felt that if these initial events can be modulated, blocked, or even accelerated, then the subsequent cascade can be altered and a diminished overall thickening could be achieved.

Some devices have been designed which permit localized delivery of a drug agent while providing enhanced perfusion capabilities. For example, one known drug delivery catheter provides an inflatable perfusion lumen which provides significantly more perfusion area than previous drug delivery devices. The disclosed catheter and method also provides drug delivery pockets on the outer periphery of the perfusion lumen. The pockets allow the drug agent to be delivered site specifically for extended periods of time.

All of the drug delivery devices discussed above, however, require that the device remain in the vessel while the drug agent is being administered. It would be desirable to have a technique for delivering a drug agent locally without the need for the drug delivery device to remain in the vessel.

To this end, some techniques have been proposed wherein a drug is delivered by a surgical procedure where a drug agent is delivered to the outside of a vessel to be treated. Studies have shown that during administration by implanting a controlled release device which surrounds the vessel (periarterial drug administration) using drugs such as heparin-ethylenevinyl acetate significantly inhibited restenosis in an arterial injury model. See for example, Edelman et al., Proc. Natl. Acad. Sci. U.S.A., 87, 3773 (1990); and Edelman et al., J. Clin. Invest., 39, 65 (1992). In these types of procedures, access to the vessel is obtained by surgically cutting to the desired location in the vessel. Then the drug agent is maintained at the desired location by wrapping a band or cuff around the vessel with the agent being loaded into the band or cuff. Although periarterial drug administration has shown some initial success in an animal model, this procedure used for delivering the implant has the obvious disadvantage of being very invasive.

Additionally, depending on the particular ailment it is known in the medical field that fluid medications can be infused directly into the wall of a vessel of a patient's cardiovascular system with beneficial results. For example, one such application involves the administration of medicaments into an arterial wall which will inhibit or prevent the restenosis of plaque in the artery. Any procedure involving the direct infusion of fluid medicaments into a vessel wall, however, requires the consideration of several factors. First, the procedure must be safe. For instance, due to the toxic nature of some medicaments, such a procedure must insure that only minimal amounts of medication are ever washed away into the blood stream and not actually infused into the vessel wall. Second, the device which infuses the medication into the vessel wall must be easy to use, accurate in its delivery capability and reliable in its operation.

Several devices have been suggested for the purpose of infusing fluid medicaments directly into a vessel wall. One type of drug delivery device is disclosed in U.S. Patent 5,538,504. This device is a catheter having a single needle bent at its distal end to define a U-shape portion. An inflatable balloon is contained within the catheter in order to deploy the needle into a vessel through a window in the catheter.

Another example of such a device is disclosed in U.S. Pat. No. 5,354,279 which issued to Hofling for an invention entitled "Plural Needle Injection Catheter". The specific device disclosed in this patent employs prebent hollow needles which are extendable from a catheter to penetrate into a vessel wall. The extended needles are then used for infusion of the fluid medicament.

U.S. Pat. No. 5,354,279 also discloses that an inner hose, which is so elastic that it can be expanded balloon-like, can be utilized to move the needles outwardly so as to engage or even pierce the surrounding vessel walls. Also, U.S. Pat. No. 5,364,356, was issued to Hofling for another invention entitled "Sleeve Catheter". This second patent to Hofling discloses a device which employs a balloon expandable sleeve that delivers fluid medication to a vessel wall. More specifically, this device of Hofling's includes a reconfigurable sleeve which is expanded by an inflatable balloon. It is intended that, as the sleeve expands, openings which are formed into the sleeve spread to discharge fluid medications onto the surface of the vessel walls.

Still another example of a device for medicating a vessel wall is disclosed in U.S. Pat. No. 5,112,305 which issued to Barath et al. for an invention entitled "Catheter Device for Intramural Delivery of Therapeutic Agents". This same device is also disclosed in a related U.S. Pat. No. 5,242,397 which issued to Barath et al. for an invention entitled "Catheter Device and Method of Use for Intramural Delivery of Protein Kinase C and Tyrosine Protein Kinase Inhibitors to Prevent Restenosis after Balloon Angioplasty". Specifically, the device disclosed by Barath et al. employs a balloon which requires an initial slow filling of the balloon with a medicament to expand the balloon and position the balloon's surface against the vessel wall. This initial slow filling is then followed by a rapid filling of the balloon which reconfigures tubular extensions on the surface of the balloon for the infusion of medicaments through the tubular extensions and into the vessel wall.

Another device for injecting fluid medication is disclosed in U.S. 5,681,281. This device is a catheter having an inflatable PET balloon mounted on a multi-lumen catheter. A plurality of injectors are mounted directly on a sleeve which is attached directly onto the outer surface of the balloon. Each injector has a base plate and a hollow protrusion projecting from the base plate. The hollow protrusion has a channel therein for pumping fluid medication into the wall of a vessel from an infusion chamber through holes in the sleeve.

To date, there have been no known devices or methods for efficiently and simultaneously delivering therapeutic agents through a plurality of penetrating sites in a controlled manner to specific portions of a vessel such as the adventitial layer.

### SUMMARY OF THE INVENTION

The present invention is directed to devices and their methods of use for delivering therapeutic agents into tissue. Although the devices and methods in accordance with the present invention can be used on any tissue requiring treatment with one or more therapeutic agents, the present invention is particularly useful for delivering one or more therapeutic agents into the wall of a vessel, and more particularly, for delivering one or more therapeutic agents in the adventitia or adventitial layer of a vessel.

In one embodiment, the present invention is directed to a device for delivering a therapeutic agent into tissue, the device comprising:
an elongated body;
a first balloon in the body expandable from a collapsed position to an expanded position;
a second balloon in the body expandable from a collapsed position to an expanded position, the second balloon having a plurality of apertures therein;
a therapeutic agent in the second balloon;
a plurality of microprojections on a surface of the second balloon for penetrating tissue; and
wherein upon expansion of the first balloon from the collapsed position to the expanded position, the second balloon is expanded from the collapsed position to the expanded position for deploying the plurality of microprojections into tissue and for dispensing the therapeutic agent from the second balloon into the tissue through the plurality of apertures.

The second balloon is a pressurized reservoir that contains the therapeutic agent therein. The second balloon is circumferentially arranged around the first balloon. And, the second balloon is located near a distal end of the elongated body of the device.

Additionally, in some embodiments, the plurality of microprojections are arranged in an array. And, the array is made from a thin sheet of material such as metal, and preferably, titanium. Moreover, the array has a plurality of openings therethrough wherein the plurality of openings are positioned adjacent the plurality of apertures in the second balloon for delivery of the therapeutic agent.

Furthermore, the device optionally includes an outer sheath that is removably positioned over the distal end of the device.
In another embodiment in accordance with the present invention, the present invention is directed to a catheter for delivering a therapeutic agent into tissue, the catheter comprising:
a flexible, elongated body;
a first balloon in the body expandable from a collapsed position to an expanded position;
a second balloon in the body expandable from a collapsed position to an expanded position, the second balloon having a plurality of apertures therein;
a therapeutic agent in the second balloon;
a plurality of microprojections on a surface of the second balloon for penetrating tissue; and
wherein upon expansion of the first balloon from the collapsed position to the expanded position, the second balloon is expanded from the collapsed position to the expanded position for deploying the plurality of microprojections into tissue and for dispensing the therapeutic agent from the second balloon into the tissue through the plurality of apertures.

The second balloon is a pressurized reservoir that contains the therapeutic agent therein. The second balloon is circumferentially arranged around the first balloon. And, the second balloon is located near a distal end of the elongated body of the catheter.

Additionally, in some embodiments, the plurality of microprojections are arranged in an array. And, the array is made from a thin sheet of material such as metal, and preferably, titanium. Moreover, the array has a plurality of openings therethrough wherein the plurality of openings are positioned adjacent the plurality of apertures in the second balloon for delivery of the therapeutic agent.

Furthermore, the catheter optionally includes an outer sheath that is removably positioned over the distal end of the catheter.

Additionally, the present invention is also directed to methods for delivering a therapeutic agent into tissue. In one embodiment according to the present invention, the method comprises the steps of:
providing a device for delivering a therapeutic agent into tissue, the device comprising: an elongated body; a first balloon in the body expandable from a collapsed position to an expanded position; a second balloon in the body expandable from a collapsed position to an expanded position, the second balloon having a plurality of apertures therein; a therapeutic agent in the second balloon; and a plurality of microprojections on a surface of the second balloon for penetrating tissue; and wherein upon expansion of the first balloon from the collapsed position to the expanded position, the second balloon is expanded from the collapsed position to the expanded position for deploying the plurality of microprojections into tissue and for dispensing the therapeutic agent from the second balloon into the tissue through the plurality of apertures;
placing the device adjacent a site in the tissue; and
delivering the therapeutic agent into the tissue at the site by expanding the first balloon from the collapsed position to the expanded position.

The method further comprises expanding the first balloon by providing a first fluid medium into the first balloon.

Another embodiment in accordance with the present invention is directed to a method for delivering a therapeutic agent into a wall of a vessel. The method comprises the steps of:
providing a device for delivering a therapeutic agent into tissue, the device comprising: an elongated body; a first balloon in the body expandable from a collapsed position to an expanded position; a second balloon in the body expandable from a collapsed position to an expanded position, the second balloon having a plurality of apertures therein; a therapeutic agent in the second balloon; and a plurality of microprojections on a surface of the second balloon for penetrating tissue; and wherein upon expansion of the first balloon from the collapsed position to the expanded position, the second balloon is expanded from the collapsed position to the expanded position for deploying the plurality of microprojections into tissue and for dispensing the therapeutic agent from the second balloon into the tissue through the plurality of apertures;
placing the device adjacent a site in the wall of the vessel; and
delivering the therapeutic agent into the wall of the vessel at the site by expanding the first balloon from the collapsed position to the expanded position.

The method further comprises expanding the first balloon by providing a first fluid medium into the first balloon. Additionally, the method further comprises placing the device within the vessel prior to delivering the therapeutic agent into the wall of the vessel.

Furthermore, the method further comprises delivering the therapeutic agent into a layer of the vessel. Particularly, the therapeutic agent is delivered into the adventitial layer of the vessel.

In another embodiment according to the present invention, the present invention is directed to a method for delivering a therapeutic agent into tissue wherein the method comprises the steps of:
providing a catheter for delivering a therapeutic agent into tissue, the catheter comprising: an elongated body; a first balloon in the body expandable from a collapsed position to an expanded position; a second balloon in the body expandable from a collapsed position to an expanded position, the second balloon having a plurality of apertures therein; a therapeutic agent in the second balloon; and a plurality of microprojections on a surface of the second balloon for penetrating tissue; and wherein upon expansion of the first balloon from the collapsed position to the expanded position, the second balloon is expanded from the collapsed position to the expanded position for deploying the plurality of microprojections into tissue and for dispensing the therapeutic agent from the second balloon into the tissue through the plurality of apertures;
placing the catheter adjacent a site in the tissue; and
delivering the therapeutic agent into the tissue at the site by expanding the first balloon from the collapsed position to the expanded position.

The method further comprises expanding the first balloon by providing a first fluid medium into the first balloon.

In another embodiment according to the present invention, the present invention is directed to a method for delivering a therapeutic agent into a wall of a vessel wherein the method comprises the steps of:
providing a catheter for delivering a therapeutic agent into tissue, the catheter comprising: an elongated body; a first balloon in the body expandable from a collapsed position to an expanded position; a second balloon in the body expandable from a collapsed position to an expanded position, the second balloon having a plurality of apertures therein; a therapeutic agent in the second balloon; and a plurality of microprojections on a surface of the second balloon for penetrating tissue; and wherein upon expansion of the first balloon from the collapsed position to the expanded position, the second balloon is expanded from the collapsed position to the expanded position for deploying the plurality of microprojections into tissue and for dispensing the therapeutic agent from the second balloon into the tissue through the plurality of apertures;
placing the catheter adjacent a site in the wall of the vessel; and
delivering the therapeutic agent into the wall of the vessel at the site by expanding the first balloon from the collapsed position to the expanded position.

The method further comprises expanding the first balloon by providing a first fluid medium into the first balloon. Additionally, the method further comprises placing the catheter within the vessel prior to delivering the therapeutic agent into the wall of the vessel. Moreover, the method further comprises delivering the therapeutic agent into a layer of the vessel. And, particularly, the method further comprises delivering the therapeutic agent into the adventitial layer of the vessel.

### BRIEF DESCRIPTION OF THE DRAWINGS

The novel features of the invention are set forth with particularity in the appended claims. The invention itself, however, both as to organization and methods of operation, together with further objects and advantages thereof, may be understood by reference to the following description, taken in conjunction with the accompanying drawings in which:
FIG. 1 is a partial perspective view of a device having microprojections for delivering a therapeutic agent in accordance with the present invention;
FIG. 2 is a view in cross-section of the distal end of the device of FIG. 1 delivering a therapeutic agent into a specific layer of tissue in accordance with the present invention; and
FIG. 3 is a schematic view of an array of microprojections for the device of FIG. 1 in accordance with the present invention.

### DESCRIPTION OF THE PREFERRED EMBODIMENTS

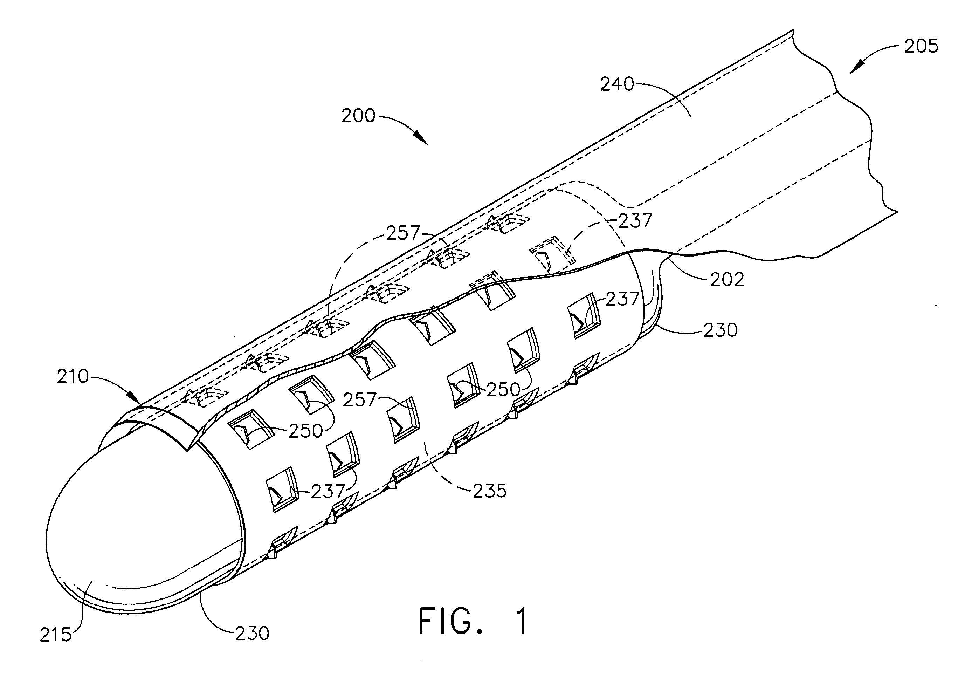

The present invention is directed to both devices and methods for delivering therapeutic agents into tissue. The device according to the present invention is generally designated 200 as best shown in FIGS. 1-3. FIG. 1 illustrates the device 200 (as a catheter) having a flexible, elongated catheter body or inner sleeve 202. As illustrated in FIG. 1, the body 202 of the catheter 200 has a distal end 210 culminating in a distal tip 215. The body 202 of catheter 200 includes both a proximal end (not shown) and a distal end 210 extending to the distal tip 215. A first expandable member or inner balloon 230, such as an inflatable balloon, is fixed to the inner sleeve or body 202 at the distal end 210 of the catheter 200. As is well understood in the field, the first expandable member or inner balloon 230 is expanded, such as through inflation with a fluid medium 400 under pressure, for example a hydraulic or pneumatic fluid, and is expandable from a collapsed or closed position or configuration to an open or expanded position or configuration.

A second expandable member or outer balloon 235 is a pressurized reservoir for therapeutic agent 500 housed or contained within this pressurized second balloon 235. The outer balloon 235 is circumferentially arranged around the inner balloon 230 and is located at the distal end 210 of the body 202 although the inner balloon 230 can be located at any desired location or portion of the body 202 so long as it is arranged with the inner balloon 230.

By way of example, the outer balloon 235 contains one or more therapeutic and/or pharmaceutical agents (drugs) 500 which exist in any fluid medium such as a liquid when housed within the outer balloon 235 or in dry form such as powder form when coated directly onto microprojections 250 and array 245 itself (if desired). The therapeutic and/or pharmaceutical agents (drugs) 500 include but are not limited to: antiproliferative/antimitotic agents including natural products such as vinca alkaloids (i.e. vinblastine, vincristine, and vinorelbine), paclitaxel, epidipodophyllotoxins (i.e. etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagine); antiplatelet agents such as G(GP)II_{b}IIIₐ inhibitors and vitronectin receptor antagonists; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nirtosoureas (carmustine (BCNU) and analogs, streptozocin), trazenes - dacarbazinine (DTIC); antiproliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine {cladribine}); platinum coordination complexes (cisplatin, carboplatin), procarbazine, hydroxyurea, mitotane, aminoglutethimide; hormones (i.e. estrogen); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); antiinflammatory: such as adrenocortical steroids (cortisol, cortisone, fludrocortisone, prednisone, prednisolone, 6α-methylprednisolone, triamcinolone, betamethasone, and dexamethasone), non- steroidal agents (salicylic acid derivatives i.e. aspirin; para-aminophenol derivatives i.e. acetominophen; indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate mofetil); angiogenic agents: vascular endothelial growth factor (VEGF), fibroblast growth factor (FGF) platelet derived growth factor (PDGF), erythropoetin,; angiotensin receptor blocker; nitric oxide donors; anti-sense oligionucleotides and combinations thereof; cell cycle inhibitors, mTOR inhibitors, and growth factor signal transduction kinase inhibitors.

Moreover, the therapeutic agents or drugs 500 are also defined to include nucleotides, nucleic acids (such as DNA and RNA), amino acids, peptides, proteins, factors, cells, extracellular matrix components such as fibers (collagen and elastin), proteoglycans, glycoproteins, lipids, etc.

Outer balloon 235 has a plurality of apertures 237 therein that permit the therapeutic agent 500 to be dispensed from the device 200 in a controlled and efficient manner as will be described in greater detail below.

Additionally, a plurality of microprojections 250 are located adjacent to apertures 237 for directly piercing and penetrating tissue to any desired depth in order to create microchannels in the pierced tissue based on the interaction of the inner balloon 230 and the outer balloon 235. Thus, as inner balloon 230 is inflated with fluid medium 400, the inner balloon 230 is expanded from its collapsed position to its expanded position. And, as inner balloon 230 is inflated and expanded, a specific ratio of the therapeutic agent 500 is dispensed from the pressurized drug reservoir or outer balloon 235 through the apertures 237 in outer balloon 235 and into the newly created microchannels formed in the pierced tissue by microprojections 250.

In accordance with the present invention, the microprojections 250 are piercing elements having a projection length less than 1000 microns. In a further embodiment, the piercing elements 250 have a projection length of less than 500 microns, more preferably, less than 250 microns. The microprojections 250 further have a width in the range of approximately 25 - 500 microns and a thickness in the range of approximately 10 - 100 microns. The microprojections may be formed in different shapes, such as needles, blades, pins, punches, and combinations thereof.

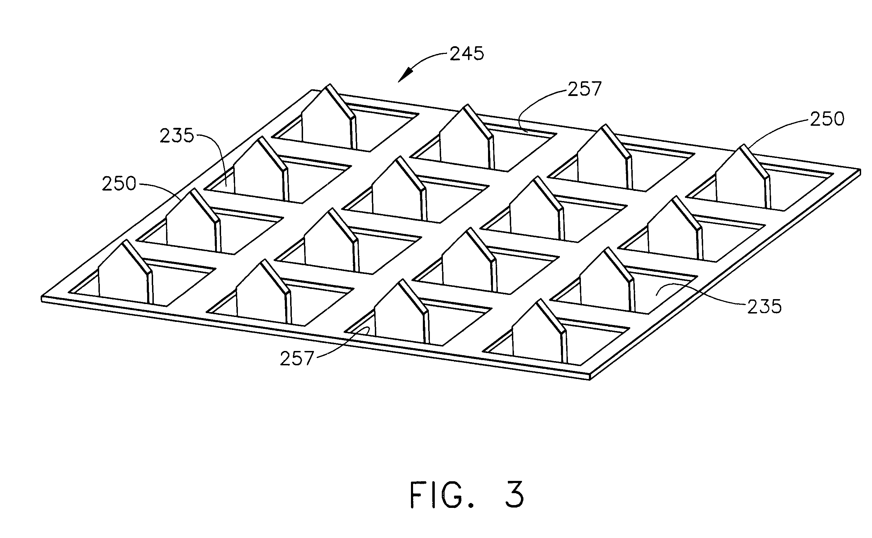

The microprojections 250 are arranged in an array 245, i.e. a microprojection array 245 comprising a plurality of microprojections 250 arranged in a specific arrangement (for example, an arrangement of rows and columns of microprojections 250) for piercing the vessel wall 300 and accessing the adventitial layer 310. The microprojection array 245 can be formed by etching or punching a plurality of microprojections 250 from a thin sheet of material (such as metal) and folding or bending the microprojections 250 out of the plane of the sheet to form a configuration, such as that shown in Fig. 3. A plurality of openings 257 are made adjacent each microprojection 250 in the sheet of array 245. The array 245 is secured to a surface of the balloon 235 such as the outer surface (or even inner surface) of balloon 235 such that openings 257 in array 245 are aligned in fluid communication with the apertures 237 in outer balloon 235. Accordingly, in this embodiment, microprojection array 245 is in the form of a thin titanium screen. The metal sheet of array 245 is not continuous so that it will be able to expand, i.e. the metal sheet of array 245 is not completely circumferentially arranged around balloon 235 or balloon 230 in order to allow expansion of balloon 235 and 230 respectively and deployment of microprojections 250 and drug 500. Alternatively, the array 245 is in the form of a stent with slots or openings that enable the stent to expand upon deployment within a vessel 300. Accordingly, Nitinol is an appropriate material for the stent so that the deployment device or balloon (can be a single balloon in this embodiment) will easily collapse and be retracted after the procedure, i.e. deployment of the stent and delivery of the drug 500 into the vessel 300. The microprojection array 245 can also be formed in other known manners, such as by forming one or more strips having microprojections along an edge of each of the strip(s) as disclosed in U.S. Patent No. 6,050,988, which is hereby incorporated by reference in its entirety.

As shown in Fig. 3, in one embodiment of a microprojection array 245 for use with the present invention, the microprojection array 245 has a plurality of microprojections 250 in a specific arrangement. And the microprojections 250 preferably extend at substantially a 90° angle from the array 245.

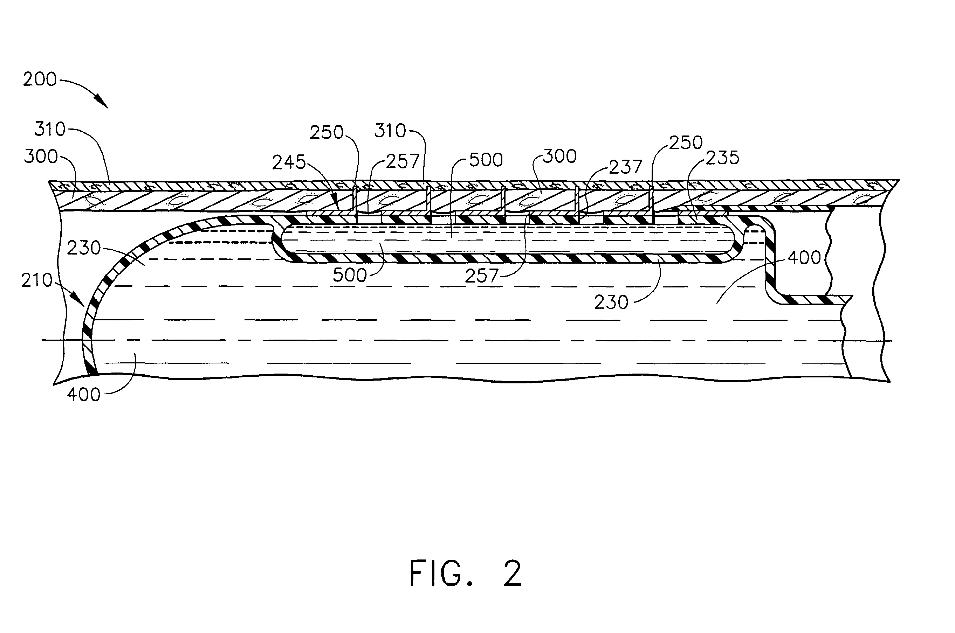

According to the invention, the array 245 may be incorporated directly onto an exterior surface of the outer balloon 235. The openings 257 in array 245 are aligned over the perforations or apertures 237 in outer balloon 235. In this embodiment, the microprojections 250 are formed by etching or punching or laser cutting a plurality of microprojections 250 from a thin metal sheet (such as a titanium sheet) or a thin film of nickel-titanium and bending the microprojections 250 out of the plane of the sheet in order to form a microprojection array 245. The array 245 can comprise any number of desired microprojections 250 which can be arranged in any number of rows and columns. By way of example, Figs. 1 and 2 illustrate an array 245 having five rows of microprojections 250 and Fig. 3 illustrating an array 245 having a four-row arrangement.

In one embodiment of the invention, the microprojection array 245 has a microprojection density of at least approximately 10 microprojections/cm², more preferably, in the range of at least approximately 50 - 2000 microprojections/cm². Preferably, the number of openings 257 per unit area through which the agent passes is at least approximately 10 openings/cm² and less than about 2000 openings/cm².

As indicated, the microprojections 250 preferably have a projection length less than 1000 microns. In one embodiment, the microprojections 250 have a projection length of less than 500 microns, more preferably, less than 250 microns. The microprojections 250 also preferably have a width in the range of approximately 25 - 500 microns and thickness in the range of approximately 10 - 100 microns.

The microprojection array 245 and microprojections 250 can be manufactured from various metals, such as stainless steel, titanium, nickel titanium alloys, or similar biocompatible materials, such as polymeric materials. Preferably, the microprojection array 245 and microprojections 250 are manufactured out of titanium.

According to the invention, the microprojection array 245 and microprojections 250 can also be constructed out of a non-conductive material, such as a polymer. Alternatively, the microprojection array 245 can be coated with a non-conductive material, such as Parylene®, or a hydrophobic material, such as Teflon®, silicon or other low energy material. The noted hydrophobic materials and associated base (e.g., photoreist) layers are set forth in U.S. Application No. 60/484,142, which is incorporated by reference herein.

Microprojection arrays 245 that can be employed with the present invention include, but are not limited to, the members disclosed in U.S. Patent Nos. 6,083,196, 6,050,988 and 6,091,975, and U.S. Pat. Pub. No. 2002/0016562, which are incorporated by reference herein in their entirety.

Other microprojection arrays 245 and microprojections 250 that can be employed with the present invention include arrays and projections formed by etching silicon using silicon chip etching techniques or by molding plastic using etched micro-molds, such as the members disclosed U.S. Patent No. 5,879,326, which is incorporated by reference herein in its entirety.

Furthermore, the microprojection 245 can also include microprojections 250 that are coated with a biocompatible coating. According to the invention, the coating can partially or completely cover each microprojection 250. For example, the coating can be in a dry pattern coating on the microprojections 250. The coating can also be applied before or after the microprojections 250 are formed. Moreover, the biocompatible coating can also contain one or more therapeutic agents or drugs 500 for delivery into the vessel wall 300 and, more particularly, for delivery directly into the adventitial layer 310 of the vessel 300.

According to the invention, the coating can be applied to the microprojections 250 by a variety of known methods. Preferably, the coating is only applied to those portions the microprojection array 245 or microprojections 250 that pierce the vessel 300.

One such coating method comprises dip-coating. Dip-coating can be described as a means to coat the microprojections by partially or totally immersing the microprojections 250 into a coating solution that includes the therapeutic agent or drug 500 (such as those described below). By use of a partial immersion technique, it is possible to limit the coating to only the tips of the microprojections 250.

A further coating method comprises roller coating, which employs a roller coating mechanism that similarly limits the coating to the tips of the microprojections 250. The roller coating method is disclosed in U.S. Application No. 10/099,604 (Pub. No. 2002/0132054), which is incorporated by reference herein in its entirety. As discussed in detail in the noted application, the disclosed roller coating method provides a smooth coating that is not easily dislodged from the microprojections 250 during piercing of the vessel 300.

An outer sheath 240, which is made of a polymer material such as polyethylene, is optionally used as a removably positionable cover for the catheter distal end 210 and serves as an additional form of protection for protecting the vessel 300 and components of the device 200 such as microprojections 250 as well as preventing any leakage of the therapeutic agent 500 from the catheter distal end 210. The cover 240 is movably positioned or movably disposed from the catheter distal end 210 in order to provide both the protection as described above as well as the unimpeded deployment of the microprojections 250 upon positioning of the microprojections 250 at its desired location at the tissue site.

The method of utilizing the catheter 200 and methods for delivering therapeutic agents 500 according to the present invention includes first identifying a location in a patient's body for delivery of therapeutic agent 500 at a number of adjacent sites in tissue. Upon identifying the desired delivery sites or locations, the catheter 200 is inserted within a vessel 300 in the patient's body. The catheter 200 is used to traverse the vessel 300 until reaching the desired location wherein the distal end 210 of the catheter 200 is positioned at the desired location within the vessel 300. The cover 240 (if used) is removed from the distal end 210 prior to expansion of the inner balloon 230. At this point, the microprojections 250 are deployed by inflating inner balloon 230 to its open or expanded the configuration by expanding or inflating with fluid medium 400.

As inner balloon 230 is inflated with fluid medium 400, the inner balloon 230 is expanded from its collapsed position to its expanded position. And, as inner balloon 230 is inflated and expanded, the microprojections 250 are advanced into the vessel 300 to a desired penetrating depth, for example, a desired layer of the vessel 300, e.g. the adventitial layer 310 thereby creating microchannels in the vessel 300 and adventitial layer 310. A specific ratio of the therapeutic agent 500 is dispensed from the pressurized drug reservoir or outer balloon 235 through the apertures 237 in outer balloon 235 and into the newly created microchannels in the vessel 300 and adventitial layer 310.

After delivery of therapeutic agent 500, inner balloon 230 is then collapsed, for instance through deflation of the expandable member, and microprojections 250 are retracted into the catheter body 202 whereby the catheter 200 is removed from the deployment site of the vessel 300 and patient's body altogether.

As best illustrated in Fig. 2, methods for delivering one or more therapeutic agents 500 using the device 200 in accordance with the present invention is shown. By way of example, the tissue of interest for receiving therapeutic treatment is a vessel 300, and more particularly, a layer within the vessel wall 300 such as the adventitial layer 310. Accordingly, once a site within the vessel 300 (on or within the vessel wall) has been identified for receiving therapeutic agent 500 at a number of distinct and adjacent perforation sites in both a controlled and simultaneous delivery manner. Thus, distal end 210 of catheter 200 is maneuvered intravenously to the vessel site 300 wherein inner balloon 230 is expanded with fluid medium 400 such that inner balloon 230 is moved by expansion from its collapsed position or collapsed configuration to its expanded position or expanded configuration. Thus, as inner balloon 230 is inflated with fluid medium 400, the outer balloon 235 serving as a pressurized reservoir for the therapeutic agent 500, is also expanded in a similar ratio or linear fashion to the expansion of inner member 230. Accordingly, array 245 of microprojections 250 are advanced outwardly and away from the longitudinal axis of distal end 210 of catheter body 202 and simultaneously pierce and penetrate vessel wall 300 with the piercing tip of each microprojection 250. As microprojections 250 are advanced into the vessel wall 300 to a desired depth or desired layer such as the adventitial layer 310, microchannels are formed in the vessel wall 300 leading and channeling therapeutic agent 500 dispensed through the apertures or perforations 237 in the outer balloon 235 as well as the openings 257 in the array 245 as inner balloon 230 is expanded or moved by inflation with fluid medium 400 from the collapsed position to the expanded position.

As inner balloon 230 is inflated or expanded at a particular inflation rate, therapeutic agent 500 is dispensed from distal end 210 of the device 200 through outer balloon 235 at a dispensing rate in a linear ratio to the inflation rate of inner balloon 230 by flowing from perforations 230 of outer balloon 235 through the openings 257 of the microprojection array 245 traveling a fluid flow path along the microchannels created in the vessel wall 300 such that the therapeutic agent 500 is channeled into the adventitial layer 310 as shown.

Accordingly, the controlled dispensing of the therapeutic agent 500 into the adventitial layer 310 of the vessel 300 is delivered simultaneously at a plurality of adjacent sites corresponding to the number of microprojections 250 on the array 245. Thus, a single application of therapeutic agent 500 with the device 200 is particularly useful for accessing and treating multiple adjacent sites in the adventitial layer 310 of vessel 300 at the same time.

Inasmuch as the foregoing specification comprises preferred embodiments of the invention, it is understood that variations and modifications may be made herein, in accordance with the inventive principles disclosed, without departing from the scope of the invention.

While preferred embodiments of the present invention have been shown and described herein, it will be obvious to those skilled in the art that such embodiments are provided by way of example only. Numerous variations, changes, and substitutions will now occur to those skilled in the art without departing from the invention. Accordingly, it is intended that the invention be limited only by the spirit and scope of the appended claims.

## Claims

1. A device for delivering a therapeutic agent into tissue, the device comprising:
an elongated body;
a first balloon in the body expandable from a collapsed position to an expanded position;
a second balloon in the body expandable from a collapsed position to an expanded position, the second balloon having a plurality of apertures therein;
a therapeutic agent in the second balloon;
a plurality of microprojections on a surface of the second balloon for penetrating tissue; and
wherein upon expansion of the first balloon from the collapsed position to the expanded position, the second balloon is expanded from the collapsed position to the expanded position for deploying the plurality of microprojections into tissue and for dispensing the therapeutic agent from the second balloon into the tissue through the plurality of apertures.

2. The device according to Claim 1, wherein the second balloon is circumferentially arranged around the first balloon.

3. The device according to Claim 1 or Claim 2, wherein the second balloon is located near a distal end of the elongated body.

4. The device according to Claim 1, Claim 2 or Claim 3, wherein the plurality of microprojections are arranged in an array.

5. The device according to Claim 4, wherein the array is made from a thin sheet or film.

6. The device according to Claim 5, wherein the array has a plurality of openings therethrough.

7. The device according to Claim 6, wherein the plurality of openings are positioned adjacent the plurality of apertures in the second balloon.

8. The device according to Claim 7, wherein the array is made from a thin sheet or film of metal.

9. The device according to Claim 8, wherein the metal is titanium or nickel-titanium.

10. The device according to any of Claims 4 to 9, wherein the second balloon and the array are located at a distal end of the body.

11. The device according to Claim 10, further comprising an outer sheath removably positioned over the distal end of the body.

12. The device according to any preceding Claim, wherein the device is a catheter.

13. The device according to Claim 12 when dependent on Claim 10, wherein the second balloon and the array are located at a distal end of the catheter.

14. The device according to any preceding Claim, wherein the body is flexible.
